# EUROPEAN PATENT SPECIFICATION

(11) **EP 2 866 897 B1**
(45) Date of publication and mention of the grant of the patent: **01.05.2019**
(21) Application number: 13737070.6
(22) Date of filing: 27.06.2013
(51) Int. Cl.: A61Q 5/12, A61Q 5/02, A61K 8/73, A61K 8/34, A61K 8/25, A61K 8/02, A61K 8/04

(54) **AEROSOL COMPOSITION COMPRISING A PARTICULATE TAPIOCA STARCH**
AEROSOLZUSAMMENSETZUNG MIT EINER BESTIMMTEN TAPIOKA-STÄRKE
COMPOSITION AÉROSOL COMPRENANT DES PARTICULES D'AMIDON DE TAPIOCA

(30) Priority: 27.06.2012 US 201261664915 P
(43) Date of publication of application: 06.05.2015
(73) Proprietor: The Procter & Gamble Company, Cincinnati, OH 45202 (US)
(72) Inventor: SWAILE, David, Frederick, Cincinnati, Ohio 45202 (US); TORRES RIVERA, Jazmin, Veronica, Cincinnati, Ohio 45202 (US); THOMAS, Michael, Edward, Cincinnati, Ohio 45202 (US)
(74) Representative: Sauvaître, Thibault Bruno
(86) International application number: PCT/US2013/048217
(87) International publication number: WO 2014/004847

(56) References cited:
- WO-A1-2011/056625
- WO-A2-2012/104362
- Akzo Nobel Personal Care: "Introducing DRY-FLO TS Starch", , 29 February 2012 (2012-02-29), XP002729870, Retrieved from the Internet: URL:http://www.sc.akzonobel.com/en/persona lcare/Pages/featured-stories-2-29-12-dfts. aspx [retrieved on 2014-09-11]

## Description

### FIELD OF THE INVENTION

The present invention relates to a method of achieving improved hair feel using an aerosol dry shampoo. More particularly, the invention relates to a method for achieving improved hair feel comprising applying an aerosol composition comprising a particulate tapioca starch.

### BACKGROUND OF THE INVENTION

Aerosol dry shampoos generally work by spraying an aerosol comprising a dry shampoo composition onto the hair. A carrier material in the dry shampoo composition evaporates and a powder remains. The powder absorbs sebum dissolved from the hair and may then fall out or be removed by brushing the hair. WO2011/056625 describes dry shampoo compositions applied as an aerosol. Akzo Nobel published an article retrievable at the web address: http://www.sc.akzonobel.com/en/personalcare/Pages/featured-stories-2-29-12-dfts.aspx entitled: "Introducing DRY-FLO TS Starch" which describes the use of a starch material in dry shampoo compositions.

However, known methods of applying aerosol dry shampoo compositions generally leave a dirty feeling to the hair. Therefore, there is a need for a method of achieving improved hair feel using an aerosol dry shampoo.

### SUMMARY OF THE INVENTION

According to one embodiment of the invention, there is provided a method of achieving improved hair feel due to a decrease in static friction comprising applying an aerosol composition to the hair with an apparatus at a spray rate from 0.4 g/sec to 0.8 g/sec, wherein said aerosol composition comprises: (a) from about 5% to about 12% particulate tapioca starch; (b) from 30% to 50% alcohol; (c) from 0.1% to 0.3% redispersing agent wherein the redispersing agent comprises silica; (d) from 40% to 60% propellant; and (e) less than about 1% nonvolatile oil; and depositing from 0.15 g to 0.35 g of nonvolatile material from said aerosol composition to the hair when said apparatus is sprayed for about 5 sec from about a 15 cm distance.

According to another embodiment of the invention, there is provided an aerosol composition comprising (a) from 5% to 12% particulate tapioca starch; (b) from 30% to 50% alcohol; (c) from 0.1% to 0.3% redispersing agent wherein the redispersing agent comprises silica; (d) from about 40% to about 60% propellant; and (e) less than 1% nonvolatile oil; wherein said particulate tapioca starch is a blend of hydrophobically modified particulate tapioca starch and unmodified particulate tapioca starch; and wherein the ratio of hydrophobically modified particulate tapioca starch to unmodified particulate tapioca starch is 2:1 or greater.

These and other features, aspects, and advantages of the present invention will become evident to those skilled in the art from a reading of the present disclosure.

### BRIEF DESCRIPTION OF THE DRAWINGS

While the specification concludes with the claims particularly pointing out and distinctly claiming the invention, it is believed that the present invention will be better understood from the following description taken in conjunction with the accompanying drawings in which:

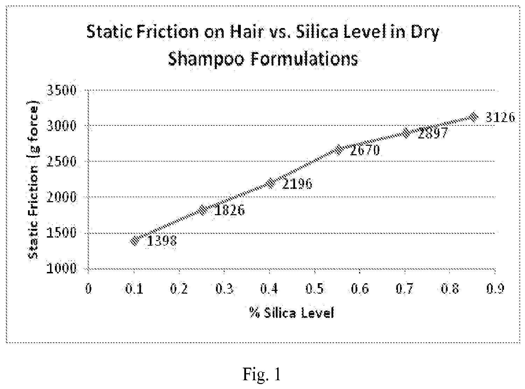

Fig. 1 shows the impact of silica level in a dry shampoo formulation on the static friction on hair.

### DETAILED DESCRIPTION OF THE INVENTION

In all embodiments of the present invention, all percentages are by weight of the total composition, unless specifically stated otherwise. All ratios are weight ratios, unless specifically stated otherwise. All ranges are inclusive and combinable. The number of significant digits conveys neither a limitation on the indicated amounts nor on the accuracy of the measurements. Unless otherwise indicated, all measurements are understood to be made at 25 °C and at ambient conditions, where "ambient conditions" means conditions under about one atmosphere of pressure and at about 50 % relative humidity. All such weights as they pertain to listed ingredients are based on the active level and do not include carriers or by-products that may be included in commercially available materials, unless otherwise specified.

The term "comprising," as used herein, means that other steps and other ingredients which do not affect the end result can be added. This term encompasses the terms "consisting of" and "consisting essentially of." The compositions and methods/processes of the present invention can comprise, consist of, and consist essentially of the elements and limitations of the invention described herein, as well as any of the additional or optional ingredients, components, steps, or limitations described herein.

The terms "include," "includes," and "including," as used herein, are meant to be nonlimiting and are understood to mean "comprise," "comprises," and "comprising," respectively.

The test methods disclosed in the Test Methods Section of the present application should be used to determine the respective values of the parameters of Applicants' inventions.

Unless otherwise noted, all component or composition levels are in reference to the active portion of that component or composition, and are exclusive of impurities, for example, residual solvents or by-products, which may be present in commercially available sources of such components or compositions.

All percentages and ratios are calculated by weight unless otherwise indicated. All percentages and ratios are calculated based on the total composition unless otherwise indicated. The term "weight percent" may be denoted as "wt.%" herein.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

The features of the method, as well as the other aspects and other relevant components, are described in detail hereinafter. All components of the composition described herein should be physically and chemically compatible with the essential components described herein, and should not otherwise unduly impair product stability, aesthetics or performance.

Formulating specific levels of particulate tapioca starch, alcohol, redispersing agent, propellant, and nonvolatile oil, can improve clean feel of the hair while maintaining the same oil-removing benefit provided by traditional compositions.

The aerosol composition comprises: (a) from 5% to 12% particulate tapioca starch; (b) from 30% to 50% alcohol; (c) from 0.1% to 0.3% redispersing agent; (d) from 40% to 60% propellant; and (e) less than 1% nonvolatile oil; wherein said aerosol composition is sprayed by an apparatus, wherein said apparatus has a spray rate from 0.4 g/sec to 0.8 g/sec, and wherein said apparatus deposits from 0.15 g to 0.35 g nonvolatile material from the aerosol composition to a surface when sprayed for about 5 sec from about a 15 cm distance.

### A. Particulate Tapioca Starch

The method for achieving improved hair feel may include applying an aerosol composition comprising a particulate tapioca starch that is dispersed rather than dissolved in the aerosol composition. The aerosol composition may comprise from about 5% to about 12% particulate tapioca starch, alternatively from 7% to 10% particulate tapioca starch, and alternatively from 8% to 9% particulate tapioca starch, by weight of the aerosol composition.

The particulate tapioca starch may be selected from the group consisting of hydrophobically modified particulate tapioca starch, hydrophobically unmodified particulate tapioca starch, and combinations thereof. A blend of particulate tapioca starch may comprise from 4% to 8% hydrophobically modified particulate tapioca starch, and from 1% to 4% unmodified particulate tapioca starch, by weight of the aerosol composition. The ratio of hydrophobically modified particulate tapioca starch to unmodified particulate tapioca starch may be 2:1 or greater.

Hydrophobically modified particulate tapioca starches may be made by a variety of methods, including those discussed in U.S. Patent No. 7,375,214, U.S. Patent No. 7,799,909, U.S. Patent No. 6,037,466, U.S. Patent No. 2,852,404, U.S. Patent No. 5,672,699, and U.S. Patent No. 5,776,476.

Modified particulate tapioca starch may be an organically modified particulate tapioca starch or a silicone grafted particulate tapioca starch. Silicone grafted particulate tapioca starch may be purchased under the trade name Dry Flo TS and under the INCI name Tapioca Starch Polymethylsilsesquioxane. Silicone modified particulate tapioca starch may be produced by a reaction of methyl sodium siliconate (polymethylsilsesquioxane) and tapioca starch. Particulate tapioca starch may be sourced from the Cassava root by standard means know in the art. One example of a commercially available silicone modified particulate tapioca starch is CAS no. 68989-12-8.

### B. Alcohol

The method for achieving improved hair feel may include applying an aerosol composition comprising an alcohol. The aerosol composition may comprise from 30% to 50% alcohol, alternatively from 31% to 40% alcohol, and alternatively from 33% to 38% alcohol, by weight of the aerosol composition.

The alcohol may be selected from the group consisting of C1-C4 monohydric alcohols. The alcohol may serve as a liquid solvent vehicle. Suitable alcohols may be selected from the group consisting of methanol, ethanol, isopropanol, and mixtures thereof.

### C. Propellant

The method for achieving improved hair feel may include applying an aerosol composition comprising a propellant. The aerosol composition may comprise from 40% to 60% propellant, alternatively from 50% to 60% propellant, and alternatively from 52% to 57% propellant, by weight of the aerosol composition.

The propellant may comprise one or more volatile materials, which in a gaseous state, may carry the other components of the aerosol composition in particulate or droplet form. The aerosol propellant may have a boiling point within the range of from -45°C to 5°C. The aerosol propellants may be liquefied when packaged in convention aerosol containers under pressure. The rapid boiling of the aerosol propellant upon leaving the aerosol container may aid in the atomization of the other components of the aerosol composition.

Aerosol propellants which may be employed in the aerosol composition may include the chemically-inert hydrocarbons such as propane, n-butane, isobutane, cyclopropane, and mixtures thereof, as well as halogenated hydrocarbons such as dichlorodiluoromethane, 1,1-dichloro-1,1,2,2-tetrafluoroethane, 1-chloro-1,1-difluoro-2,2-trifluroethane, 1-chloro-1,1-difluoroethylene, 1,1-difluoroethane, dimethyl ether, monochlorodifluoromethane, trans-1,3,3,3-tetrafluoropropene , and mixtures thereof. The propellant may comprise hydrocarbons such as isobutane, propane, and butane - these materials may be used for their low ozone reactivity and may be used as individual components where their vapor pressures at 21.1°C range from about 1.17 Bar to about 7.45 Bar, alternatively from about 1.17 Bar to about 4.83 Bar, and alternatively from about 2.14 Bar to about 3.79 Bar. Suitable propellants include, but are not limited to, propellants that may be miscible (soluble) with the alcohol in the aerosol composition.

### D. Redispersing Agent

The method for achieving improved hair feel may include applying an aerosol composition comprising a redispersing agent to help suspend any dispersed solids or liquids within the composition. Including a redispersing agent in the aerosol composition may separate the tapioca starch particles upon settling between uses, thereby preventing the creation of resilient particle aggregates that cannot be broken with reasonable shaking.

Referring to Figure 1, Applicants have surprisingly found that having a specific level of redispersing agent allows for improved hair feel due to a decrease in static friction - the lower the silica level in the aerosol composition, the lower the static friction on the hair. Figure 1 shows the impact of silica level in a dry shampoo on the static friction on hair. The aerosol composition may comprise from 0.1% to 0.3% redispersing agent, by weight of the aerosol composition.

### Static Friction Test Method (IFF Method)

The inter-fiber friction method emulates the motion of rubbing hair between the thumb and index finger in an up and down direction. Inter-fiber Friction (IFF) evaluates the hair to hair interaction of dried hair tresses, providing a "dry hair feel" measure. An Instron or Texture Analyzer measures hair to hair interaction (resistance/static friction) in both directions while applying a constant pressure to a hair switch, sandwiched between artificial skin surrogates. Friction is the opposing, resistive force between two material surfaces that acts to hinder relative motion between them. Static friction is the force of friction that exists between two solid surfaces that are non-moving.

Suitable redispersing agents may include, but are not limited to, any material known or otherwise effective in providing suspending or bulking properties to the composition, or which otherwise provide the desired viscosity to the final product form. The redispersing agent may be insoluble in the aerosol composition. The redispersing agent may be a hydrophilic redispersing agent selected from the group consisting of silica, clays, and mixtures thereof. Suitable hydrophilic silica particles include, but are not limited to, hydrophilic fumed silica particles. The hydrophilic fumed silica particles may each have a total surface area of greater than 100 m²/g. Examples of commercialized silica from Evonik Corporation include Aerosil® 200, Aerosil® 300, Aerosil® R972, and Aerosil® 812. Examples of commercialized silica from Cabot Corporation include CAB-O-SIL® H-5 and CAB-O-SIL® M-5.

Suitable redispersing agents include, but are not limited to hydrophilic clay particles. Examples of commercialized clay from Southern Clay Corporation include Laponite® XLG, Laponite® XLS, Laponite® XL31, and Laponite® D. Other suitable clay particles may include hydrophilic hectorite, laponite, and bentonite clays.

### E. Nonvolatile Oil

The method for achieving improved hair feel may include applying an aerosol composition comprising a nonvolatile oil. Nonvolatile oil may be used to increase the substantivity of the particulate tapioca starch and/or other benefit agents. The aerosol composition may comprise less than 1% nonvolatile oil, alternatively less than 0.5% nonvolatile oil, alternatively less than 0,25% nonvolatile oil, and alternatively about 0% nonvolatile oil, by weight of the aerosol composition. Inclusion of a nonvolatile oil above 1% by weight of the aerosol composition may decrease absorption characteristics of the particulate starch and the clean feel of the hair.

Suitable nonvolatile oils include, but are not limited to, linear silicones with viscosity values of up to about 100,000 centistoke. The nonvolatile oil may be selected from the group consisting of nonvolatile polar organic solvents such as mono and polyhydric alcohols, fatty mono and polyhydric alcohols, fatty acids, esters of mono and dibasic carboxylic acids with mono and polyhydric alcohols, polyoxyethylenes, polyoxypropylenes, polyalkoxylates ethers of alcohols, and combinations thereof.

The nonvolatile oil may be a water-immiscible liquid under ambient conditions. Specific nonlimiting examples of such nonvolatile oils include propyleneglycol monoisostearate, PPG-3 myristyl ether, PEG-8, 1,2, pentanediol, PPG-14 butylether, dimethyl isosorbide, isopropyl myristate, ethyl laurate, isopropyl palmitate, isopropyl behenate, decyl acetate, behenyl butyrate, hexadecyl acetate, decyl decanoate, methyl oleate, lauryl laurate, dioctyladipate, and combinations thereof. Other suitable nonvolatile oils which may be used herein are described in Cosmetics, Science, and Technology, Vol. 1, 27-104, edited by Balsam and Sagarin (1972).

### F. Spray Rate

The method for achieving improved hair feel may include applying an aerosol composition wherein said aerosol composition is sprayed by an apparatus; and wherein the apparatus has a spray rate. The apparatus may be any apparatus suitable for spraying an aerosol composition. The spray rate may be from 0.4 g/sec to 0.8 g/sec, alternatively from 0.5 g/sec to 0.7 g/sec.

### Spray Rate Test Method

### 1. Equipment

a. Balance capable of weighing to the nearest .01 of a gram.
b. Water bath equipped with automatic heater and chiller capable of operating at 21 ± 1 °C.
c. Hood.
d. Stopwatch or clock with accuracy to 0.1 second.

### 2. Test Method

a. Remove covercaps. Uniquely mark each unit.
b. Place units in the controlled water bath for about 30 minutes ± 3. Remove one can at a time for spraying. Handle as little as possible and or wear gloves to remove the effect the body temperature has on the test.
c. Shake unit well if shaking is specified on can copy, otherwise do not shake. Actuate unit briefly (∼1 second).
d. Weigh the unit and re-shake if shaking is specified.
e. Holding the can upright, actuate the unit with full product flow for a 10.00 ± 0.1 second actuation period. Weigh the unit. Be sure the actuation is for full valve opening and that the valve is depressed vertically. A stopwatch as defined above shall be used. A mechanical spray testing device can be used.
f. During actuation, if required determine if spray pattern is uniform and similar to the required production standard.
g. Calculate spray rate in grams/second for the 10 second actuation.
h. Test each unit only once.

### G. Deposition

The method for achieving improved hair feel may include applying an aerosol composition wherein said aerosol composition is sprayed by an apparatus; and the apparatus deposits a certain amount of nonvolatile material from the aerosol composition. The nonvolatile material from the aerosol composition includes the particulate tapioca starch, the redispersing agent, any additional benefit agent and/or non volatile oil, and any other nonvolatile materials that may be added to the aerosol composition. The apparatus may deposit from 0.15 g to 0.35 g nonvolatile material to a surface when sprayed for about 5 sec from about a 15 cm distance, alternatively from 0.2 g to 0.3 g nonvolatile material to a surface when sprayed for about 5 sec from about a 15 cm distance.

### Deposition Test Method

1. Place product in water bath at room temperature (21.1°C) for 5 minutes.
2. Remove product from water bath and dry.
3. Shake product vigorously by hand for 10 seconds using vertical and side-to-side motion to ensure uniform distribution of concentrate and propellant in can.
4. Record initial weight of deposition material (*y₁*).
5. Spray for 5 seconds onto deposition material from 6".
6. Allow the deposition material to dry for 60 minutes in an open room and record weight (*z*).
7. The total nonvolatile material deposited = *z* - *y₁.*

### H. Additional Benefit Agents

The aerosol composition may further comprise one or more additional benefit agents. Suitable benefit agents may be selected from the group consisting of anti-dandruff agents, vitamins, lipid soluble vitamins, chelants, perfumes, brighteners, enzymes, sensates, attractants, anti-bacterial agents, dyes, pigments, bleaches, and mixtures thereof.

The aerosol composition may comprise from 0.1% to 1% perfume, and alternatively from 0.1% to 0.3% perfume.

### a. Anti-Dandruff Agent

The aerosol composition may comprise an anti-dandruff agent, which may be an anti-dandruff active particulate. Such anti-dandruff particulate should be physically and chemically compatible with the components of the composition, and should not otherwise unduly impair product stability, aesthetics or performance.

Suitable anti-dandruff agents may be selected from the group consisting of: pyridinethione salts; azoles, such as ketoconazole, econazole, and elubiol; selenium sulphide; particulate sulfur; keratolytic agents such as salicylic acid; and mixtures thereof.

Pyridinethione salts may be suitable anti-dandruff active particulates. The anti-dandruff active may be a 1-hydroxy-2-pyridinethione salt in particulate form. The concentration of pyridinethione anti-dandruff particulate may range from 0.01 wt% to 5 wt%, or from 0.1 wt% to 3 wt%, or from 0.1 wt% to 2 wt%. The pyridinethione salts include those formed from heavy metals such as zinc, tin, cadmium, magnesium, aluminium and zirconium, generally zinc, typically the zinc salt of 1-hydroxy-2-pyridinethione (known as "zinc pyridinethione" or "ZPT"), commonly 1-hydroxy-2-pyridinethione salts in platelet particle form. The 1-hydroxy-2-pyridinethione salts in platelet particle form have an average particle size of up to 20 microns, or up to 5 microns, or up to 2.5 microns. Salts formed from other cations, such as sodium, may also be suitable. Pyridinethione anti-dandruff actives are described, for example, in U.S. Pat. No. 2,809,971; U.S. Pat. No. 3,236,733; U.S. Pat. No. 3,753,196; U.S. Pat. No. 3,761,418; U.S. Pat. No. 4,345,080; U.S. Pat. No. 4,323,683; U.S. Pat. No. 4,379,753; and U.S. Pat. No. 4,470,982.

In addition to the anti-dandruff active selected from polyvalent metal salts of pyrithione, the solution may further comprise one or more anti-fungal and/or anti-microbial actives. The anti-microbial active may be selected from the group consisting of coal tar, sulfur, charcoal, whitfield's ointment, castellani's paint, aluminum chloride, gentian violet, octopirox (piroctone olamine), ciclopirox olamine, undecylenic acid and its metal salts, potassium permanganate, selenium sulphide, sodium thiosulfate, propylene glycol, oil of bitter orange, urea preparations, griseofulvin, 8-hydroxyquinoline ciloquinol, thiobendazole, thiocarbamates, haloprogin, polyenes, hydroxypyridone, morpholine, benzylamine, allylamines (such as terbinafine), tea tree oil, clove leaf oil, coriander, palmarosa, berberine, thyme red, cinnamon oil, cinnamic aldehyde, citronellic acid, hinokitol, ichthyol pale, Sensiva SC-50, Elestab HP-100, azelaic acid, lyticase, iodopropynyl butylcarbamate (IPBC), isothiazalinones such as octyl isothiazalinone, and azoles, and mixtures thereof. The anti-microbial may also be selected from the group consisting of itraconazole, ketoconazole, selenium sulphide, coal tar, and mixtures thereof.

The azole anti-microbials may be an imidazole selected from the group consisting of: benzimidazole, benzothiazole, bifonazole, butaconazole nitrate, climbazole, clotrimazole, croconazole, eberconazole, econazole, elubiol, fenticonazole, fluconazole, flutimazole, isoconazole, ketoconazole, lanoconazole, metronidazole, miconazole, neticonazole, omoconazole, oxiconazole nitrate, sertaconazole, sulconazole nitrate, tioconazole, thiazole, and mixtures thereof, or the azole anti-microbials is a triazole selected from the group consisting of: terconazole, itraconazole, and mixtures thereof. When present in the hair care composition, the azole anti-microbial active may be included in an amount of from 0.01 wt% to 5 wt%, or from 0.1 wt% to 3 wt%, or from 0.3 wt% to 2 wt%. The sole anti-microbial active may be ketoconazole.

The aerosol composition may also comprise a combination of anti-microbial actives. The combination of anti-microbial actives may be selected from the group of combinations consisting of: octopirox and zinc pyrithione, pine tar and sulfur, salicylic acid and zinc pyrithione, salicylic acid and elubiol, zinc pyrithione and elubiol, zinc pyrithione and climbasole, octopirox and climbasole, salicylic acid and octopirox, and mixtures thereof.

The aerosol composition may comprise an effective amount of a zinc-containing layered material. The composition may comprise from 0.001 wt% to 10 wt%, or from about 0.01 wt% to 7 wt%, or from 0.1 wt% to 5 wt% of a zinc-containing layered material, by total weight of the composition.

Zinc-containing layered materials may be those with crystal growth primarily occurring in two dimensions. It is conventional to describe layered structures as not only those in which all the atoms are incorporated in well-defined layers, but also those in which there are ions or molecules between the layers, called gallery ions (A.F. Wells "Structural Inorganic Chemistry" Clarendon Press, 1975). Zinc-containing layered materials (ZLMs) may have zinc incorporated in the layers and/or be components of the gallery ions. The following classes of ZLMs represent relatively common examples of the general category and are not intended to be limiting as to the broader scope of materials which fit this definition.

Many ZLMs occur naturally as minerals. The ZLM may be selected from the group consisting of: hydrozincite (zinc carbonate hydroxide), aurichalcite (zinc copper carbonate hydroxide), rosasite (copper zinc carbonate hydroxide), and mixtures thereof. Related minerals that are zinc-containing may also be included in the composition. Natural ZLMs can also occur wherein anionic layer species such as clay-type minerals (e.g., phyllosilicates) contain ion-exchanged zinc gallery ions. All of these natural materials can also be obtained synthetically or formed *in situ* in a composition or during a production process.

Another common class of ZLMs, which are often, but not always, synthetic, is layered double hydroxides. The ZLM may be a layered double hydroxide conforming to the formula [M²⁺₁₋ₓM³⁺ₓ(OH)₂]^{x+} A^{m-}_{x/m}·nH₂O wherein some or all of the divalent ions (M²⁺) are zinc ions (Crepaldi, EL, Pava, PC, Tronto, J, Valim, JB J. Colloid Interfac. Sci. 2002, 248, 429-42).

Yet another class of ZLMs can be prepared called hydroxy double salts (Morioka, H., Tagaya, H., Karasu, M, Kadokawa, J, Chiba, K Inorg. Chem. 1999, 38, 4211-6). The ZLM may be a hydroxy double salt conforming to the formula [M²⁺₁₋ₓM²⁺₁₊ₓ(OH)_{3(1-y)}]⁺ Aⁿ⁻_{(1=3y)/n}·nH₂O where the two metal ions (M²⁺) may be the same or different. If they are the same and represented by zinc, the formula simplifies to [Zn₁₊ₓ(OH)₂]^{2x+} 2x A⁻·nH₂O. This latter formula represents (where x=0.4) materials such as zinc hydroxychloride and zinc hydroxynitrate. The ZLM may be zinc hydroxychloride and/or zinc hydroxynitrate. These are related to hydrozincite as well wherein a divalent anion replace the monovalent anion. These materials can also be formed *in situ* in a composition or in or during a production process.

In aerosol compositions having a zinc-containing layered material and a pyrithione or polyvalent metal salt of pyrithione, the ratio of zinc-containing layered material to pyrithione or a polyvalent metal salt of pyrithione is from 5:100 to 10:1, or from 2:10 to 5:1, or from 1:2 to 3:1.

The on-scalp deposition of the anti-dandruff active may be at least about 1 microgram/cm². The on-scalp deposition of the anti-dandruff active is important in view of ensuring that the anti-dandruff active reaches the scalp where it is able to perform its function. The deposition of the anti-dandruff active on the scalp may be at least 1.5 microgram/cm², or at least 2.5 microgram/cm², or at least 3 microgram/cm², or at least 4 microgram/cm², or at least 6 microgram/cm², or at least 7 microgram/cm², or at least 8 microgram/cm², or at least 8 microgram/cm², or at least 10 microgram/cm². The on-scalp deposition of the anti-dandruff active may be measured by having the hair of individuals washed with a composition comprising an anti-dandruff active by a trained cosmetician according to a conventional washing protocol. The hair is then parted on an area of the scalp to allow an open-ended glass cylinder to be held on the surface while an aliquot of an extraction solution is added and agitated prior to recovery and analytical determination of anti-dandruff active content by conventional methodology, such as HPLC.

### I. Examples

The following examples in Tables 1 and 2 are representative of the present invention. The exemplified compositions may be prepared by conventional formulation and mixing techniques. It will be appreciated that other modifications of the aerosol composition within the skill of those in the art may be undertaken without departing from the spirit and scope of this invention. All parts, percentages, and ratios herein are by weight unless otherwise specified. Some components may come from suppliers as dilute solutions. The amount stated reflects the weight percent of the active material, unless otherwise specified.

**Table 1**

| Ingredient | Wt.% |
|---|---|
| Isobutane, Propane, and Butane | 55.00 |
| SDA 40B Ethanol 200 Proof | 36.88 |
| Tapioca Starch Polymethylsilsesquioxane | 6.01 |
| Tapioca Starch | 1.76 |
| SILICA 200 m²/g | 0.15 |
| ROYAL HUE LC | 0.14 |
| PANTHENOL | 0.03 |
| Panthenyl Ethyl Ether | 0.03 |

**Table 2**

| Ingredient | Wt.% |
|---|---|
| Isobutane, Propane, and Butane | 55.00 |
| SDA 40B Ethanol 200 Proof | 36.78 |
| Tapioca Starch Polymethylsilsesquioxane | 6.01 |
| Tapioca Starch | 1.76 |
| SILICA 200 m²/g | 0.25 |
| ROYAL HUE LC | 0.14 |
| PANTHENOL | 0.03 |
| Panthenyl Ethyl Ether | 0.03 |

## Claims

1. A method of achieving improved hair feel due to a decrease in static friction comprising:
a. applying an aerosol composition to the hair with an apparatus at a spray rate from 0.4 g/sec to 0.8 g/sec, wherein said aerosol composition comprises:
i. from 5% to 12% particulate tapioca starch;
ii. from 30% to 50% alcohol;
iii. from 0.1% to 0.3% redispersing agent;
iv. from 40% to 60% propellant; and
v. less than 1% nonvolatile oil
wherein the percentages of (i) - (v) are by weight of the aerosol composition, wherein the redispersing agent comprises silica; and
b. depositing from 0.15 g to 0.35 g of nonvolatile material from said aerosol composition to the hair when said apparatus is sprayed for 5 sec from a 15 cm distance.

2. The method of Claim 1, wherein said particulate tapioca starch is a blend of hydrophobically modified particulate tapioca starch and unmodified particulate tapioca starch.

3. The method of Claim 2, wherein the ratio by weight of hydrophobically modified particulate tapioca starch to unmodified particulate tapioca starch is 2:1 or greater.

4. The method of Claims 2 or 3, wherein said aerosol composition comprises from 4% to 8% hydrophobically modified particulate tapioca starch, by weight of the aerosol composition.

5. The method of Claims 2, 3, or 4, wherein said aerosol composition comprises from 1% to 4% unmodified particulate tapioca starch, by weight of the aerosol composition.

6. The method according to any preceding claims, wherein said aerosol composition comprises from 5% to 12% particulate tapioca starch, more preferably from 7% to 10% particulate tapioca starch, by weight of the aerosol composition.

7. The method according to any preceding claims, wherein said redispersing agent is fumed silica.

8. The method according to any preceding claims 1, wherein said aerosol composition comprises from 33% to 38% alcohol, by weight of the aerosol composition.

9. The method according to any preceding claims, wherein said aerosol composition comprises from 52% to 57% propellant, by weight of the aerosol composition.

10. The method according to any preceding claims, wherein said aerosol composition further comprises one or more additional benefit agents.

11. The method of Claim 10, wherein said one or more additional benefit agents is selected from the group consisting of anti-dandruff agents, vitamins, chelants, perfumes, brighteners, enzymes, sensates, attractants, anti-bacterial agents, dyes, pigments, bleaches, and mixtures thereof.

12. The method according to any preceding claims, wherein said aerosol composition comprises from 0.1% to 1% perfume, more preferably from 0.1% to 0.3% perfume, by weight of the aerosol composition.

13. The method according to any preceding claims, wherein said apparatus deposits from 0.2 g to 0.3 g nonvolatile material to a surface when sprayed for 5 sec from a 15 cm distance.

14. The method according to any preceding claims, wherein said apparatus has a spray rate from 0.5 g/sec to 0.7 g/sec.

15. An aerosol composition comprising:
i. from 5% to 12% particulate tapioca starch;
ii. from 30% to 50% alcohol;
iii. from 0.1% to 0.3% redispersing agent;
iv. from 40% to 60% propellant; and
v. less than 1% nonvolatile oil;
wherein the percentages of (i) - (v) are by weight of the aerosol composition, wherein the redispersing agent comprises silica; and
wherein said particulate tapioca starch is a blend of hydrophobically modified particulate tapioca starch and unmodified particulate tapioca starch; and
wherein the ratio of hydrophobically modified particulate tapioca starch to unmodified particulate tapioca starch is 2:1 or greater.

## Patentansprüche

1. Verfahren zum Erreichen eines verbesserten Haargefühls aufgrund einer Abnahme der Haftreibung, umfassend:
a. Auftragen einer Aerosolzusammensetzung auf das Haar mit einem Apparat mit einer Sprührate von 0,4 g/s bis 0,8 g/s, wobei die Aerosolzusammensetzung umfasst:
i. von 5 % bis 12 % teilchenförmige Tapiokastärke;
ii. von 30 % bis 50 % Alkohol;
iii. von 0,1 % bis 0,3 % Redispergiermittel;
iv. von 40 % bis 60 % Treibmittel; und
v. weniger als 1 % nichtflüchtiges Öl;
wobei die Prozentsätze von (i) bis (v) auf das Gewicht der Aerosolzusammensetzung bezogen sind, wobei das Redispergiermittel Silica umfasst; und
b. Abscheiden von 0,15 g bis 0,35 g von nichtflüchtigem Material von der Aerosolzusammensetzung auf das Haar, wenn mit dem Apparat für 5 Sekunden aus einem Abstand von 15 cm gesprüht wird.

2. Verfahren nach Anspruch 1, wobei die teilchenförmige Tapiokastärke eine Mischung aus hydrophob modifizierter, teilchenförmiger Tapiokastärke und unmodifizierter, teilchenförmiger Tapiokastärke ist.

3. Verfahren nach Anspruch 2, wobei das Gewichtsverhältnis von hydrophob modifizierter, teilchenförmiger Tapiokastärke zu unmodifizierter, teilchenförmiger Tapiokastärke 2:1 oder größer ist.

4. Verfahren nach Anspruch 2 oder 3, wobei die Aerosolzusammensetzung von 4 Gew.-% bis 8 Gew.-% hydrophob modifizierte, teilchenförmige Tapiokastärke, bezogen auf das Gewicht der Aerosolzusammensetzung, umfasst.

5. Verfahren nach Anspruch 2, 3 oder 4, wobei die Aerosolzusammensetzung von 1 Gew.-% bis 4 Gew.-% unmodifizierte, teilchenförmige Tapiokastärke, bezogen auf das Gewicht der Aerosolzusammensetzung, umfasst.

6. Verfahren nach einem der vorstehenden Ansprüche, wobei die Aerosolzusammensetzung von 5 Gew.-% bis 12 Gew.-% teilchenförmige Tapiokastärke, mehr bevorzugt von 7 Gew.-% bis 10 Gew.-% teilchenförmige Tapiokastärke, bezogen auf das Gewicht der Aerosolzusammensetzung, umfasst.

7. Verfahren nach einem der vorstehenden Ansprüche, wobei das Redispergiermittel pyrogene Kieselsäure ist.

8. Verfahren nach einem der vorstehenden Ansprüche, wobei die Aerosolzusammensetzung von 33 Gew.-% bis 38 Gew.-% Alkohol, bezogen auf das Gewicht der Aerosolzusammensetzung, umfasst.

9. Verfahren nach einem der vorstehenden Ansprüche, wobei die Aerosolzusammensetzung von 52 Gew.-% bis 57 Gew.-% Treibmittel, bezogen auf das Gewicht der Aerosolzusammensetzung, umfasst.

10. Verfahren nach einem der vorstehenden Ansprüche, wobei die Aerosolzusammensetzung ferner einen oder mehrere zusätzliche Wirkstoffe umfasst.

11. Verfahren nach Anspruch 10, wobei der eine oder die mehreren zusätzlichen Wirkstoffe ausgewählt sind aus der Gruppe bestehend aus Antischuppenmitteln, Vitaminen, Chelants, Duftstoffen, Aufhellern, Enzymen, sinnlich Wahrgenommenem, Lockstoffen, antibakteriellen Mitteln, Farbstoffen, Pigmenten, Bleichmitteln und Mischungen davon.

12. Verfahren nach einem der vorstehenden Ansprüche, wobei die Aerosolzusammensetzung von 0,1 Gew.-% bis 1 Gew.-% Duftstoff, mehr bevorzugt von 0,1 Gew.-% bis 0,3 Gew.-% Duftstoff, bezogen auf das Gewicht der Aerosolzusammensetzung, umfasst.

13. Verfahren nach einem der vorstehenden Ansprüche, wobei der Apparat von 0,2 g bis 0,3 g nichtflüchtiges Material auf eine Oberfläche abscheidet, wenn für 5 Sekunden aus einem Abstand von 15 cm gesprüht wird.

14. Verfahren nach einem der vorstehenden Ansprüche, wobei der Apparat eine Sprührate von 0,5 g/s bis 0,7 g/s aufweist.

15. Aerosolzusammensetzung, umfassend:
i. von 5 % bis 12 % teilchenförmige Tapiokastärke;
ii. von 30 % bis 50 % Alkohol;
iii. von 0,1 % bis 0,3 % Redispergiermittel;
iv. von 40 % bis 60 % Treibmittel; und
v. weniger als 1 % nichtflüchtiges Öl;
wobei die Prozentsätze von (i) bis (v) auf das Gewicht der Aerosolzusammensetzung bezogen sind, wobei das Redispergiermittel Silica umfasst; und
wobei die teilchenförmige Tapiokastärke eine Mischung aus hydrophob modifizierter, teilchenförmiger Tapiokastärke und unmodifizierter, teilchenförmiger Tapiokastärke ist; und
wobei das Verhältnis von hydrophob modifizierter, teilchenförmiger Tapiokastärke zu unmodifizierter, teilchenförmiger Tapiokastärke 2:1 oder größer ist.

## Revendications

1. Procédé pour obtenir une texture de cheveu améliorée du fait d'une diminution du frottement statique, comprenant :
a. l'application d'une composition d'aérosol sur les cheveux avec un appareil à un débit de pulvérisation allant de 0,4 g/s à 0,8 g/s, dans lequel ladite composition d'aérosol comprend :
i. de 5 % à 12 % d'amidon de tapioca particulaire ;
ii. de 30 % à 50 % d'alcool ;
iii. de 0,1 % à 0,3 % d'agent de redispersion ;
iv. de 40 % à 60 % de propulseur ; et
v. moins de 1 % d'huile non volatile ;
dans laquelle les pourcentages des points (i) à (v) sont exprimés en poids de la composition d'aérosol, dans laquelle l'agent de redispersion comprend de la silice ; et
b. le dépôt de 0,15 g à 0,35 g de matériau non volatil à partir de ladite composition d'aérosol sur les cheveux lorsque ledit appareil pulvérise pendant 5 s à une distance de 15 cm.

2. Procédé selon la revendication 1, dans lequel ledit amidon de tapioca particulaire est un mélange d'amidon de tapioca particulaire rendu hydrophobe et d'amidon de tapioca particulaire non modifié.

3. Procédé selon la revendication 2, dans lequel le rapport en poids de l'amidon de tapioca particulaire rendu hydrophobe à l'amidon de tapioca particulaire non modifié est de 2:1 ou plus.

4. Procédé selon les revendications 2 ou 3, dans lequel ladite composition d'aérosol comprend de 4 % à 8 % d'amidon de tapioca particulaire rendu hydrophobe, en poids de la composition d'aérosol.

5. Procédé selon les revendications 2, 3 ou 4, dans lequel ladite composition d'aérosol comprend de 1 % à 4 % d'amidon de tapioca particulaire non modifié, en poids de la composition d'aérosol.

6. Procédé selon l'une quelconque des revendications précédentes, dans lequel ladite composition d'aérosol comprend de 5 % à 12 % d'amidon de tapioca particulaire, plus préférablement de 7 % à 10 % d'amidon de tapioca particulaire, en poids de la composition d'aérosol.

7. Procédé selon l'une quelconque des revendications précédentes, dans lequel ledit agent de redispersion est de la silice pyrogénée.

8. Procédé selon l'une quelconque des revendications précédentes, dans lequel ladite composition d'aérosol comprend de 33 % à 38 % d'alcool, en poids de la composition d'aérosol.

9. Procédé selon l'une quelconque des revendications précédentes, dans lequel ladite composition d'aérosol comprend de 52 % à 57 % de propulseur, en poids de la composition d'aérosol.

10. Procédé selon l'une quelconque des revendications précédentes, dans lequel ladite composition d'aérosol comprend en outre un ou plusieurs agents bénéfiques supplémentaires.

11. Procédé selon la revendication 10, dans lequel lesdits un ou plusieurs agents bénéfiques supplémentaires sont choisis dans le groupe constitué des agents antipelliculaires, des vitamines, des agents chélatants, des parfums, des azurants, des enzymes, des sensates, des attractifs, des agents antibactériens, des teintures, des pigments, des agents de blanchiment et des mélanges de ceux-ci.

12. Procédé selon l'une quelconque des revendications précédentes, dans lequel ladite composition d'aérosol comprend de 0,1 % à 1 % de parfum, plus préférablement de 0,1 % à 0,3 % de parfum, en poids de la composition d'aérosol.

13. Procédé selon l'une quelconque des revendications précédentes, dans lequel ledit appareil dépose de 0,2 g à 0,3 g de matériau non volatil sur une surface lorsqu'il pulvérise pendant 5 s à une distance de 15 cm.

14. Procédé selon l'une quelconque des revendications précédentes, dans lequel ledit appareil a un débit de pulvérisation de 0,5 g/s à 0,7 g/s.

15. Composition d'aérosol comprenant :
i. de 5 % à 12 % d'amidon de tapioca particulaire ;
ii. de 30 % à 50 % d'alcool ;
iii. de 0,1 % à 0,3 % d'agent de redispersion ;
iv. de 40 % à 60 % de propulseur ; et
v. moins de 1 % d'huile non volatile ;
dans laquelle les pourcentages des points (i) à (v) sont exprimés en poids de la composition d'aérosol, dans laquelle l'agent de redispersion comprend de la silice ; et
dans laquelle ledit amidon de tapioca particulaire est un mélange d'amidon de tapioca particulaire rendu hydrophobe et d'amidon de tapioca particulaire non modifié ; et
dans laquelle le rapport de l'amidon de tapioca particulaire rendu hydrophobe à l'amidon de tapioca particulaire non modifié est de 2:1 ou plus.
